# EUROPEAN PATENT SPECIFICATION

(11) **EP 1 666 073 B1**
(45) Date of publication and mention of the grant of the patent: **27.08.2008**
(21) Application number: 05019049.5
(22) Date of filing: 01.09.2005
(51) Int. Cl.: A61L 2/00, A61L 2/22, A61L 2/26, A61K 31/40, A61K 31/60, B05B 7/00, A01N 25/16, A47K 5/14, A61K 8/49

(54) **Device for delivering an antimicrobial composition**
Vorrichtung zur Abgabe einer antimikrobiellen Zusammensetzung
Dispositif pour la délivrance d'une composition antimicrobienne

(30) Priority: 13.10.2004 EP 04256299
(43) Date of publication of application: 07.06.2006
(73) Proprietor: The Procter and Gamble Company, Cincinnati, Ohio 45202 (US)
(72) Inventor: Rennie, Paul John, Surrey GU7 2HA (GB); Simpson, Michael Alan, Surrey TW20 0RW (GB); Sreedharan, Sanchy, Surrey KT15 1UR (GB)
(74) Representative: Clemo, Nicholas Graham

(56) References cited:
- EP-A- 0 990 412
- WO-A-01/28552
- US-A- 5 232 632

## Description

### FIELD OF THE INVENTION

The present invention is related to the field of anti-microbial devices, more specifically anti-microbial compositions and packages for containing and dispensing them as a foam. The devices herein deliver anti-microbial compositions which provide enhanced immediate as well as residual anti-viral and antibacterial efficacy and improved efficiency of coverage when used.

### BACKGROUND OF THE INVENTION

Human health is impacted by a variety of microbial organisms. Inoculation of humans or other mammals by these microorganisms often results in various sicknesses and ailments. Public awareness of such contaminations has been heightened due to the increased number of food poisonings, streptococcal infections, etc. which have been occurring in the recent past. Consequently, there has been a thrust by the medical community to persuade the general public to wash any areas which generally come in contact with infected surfaces like body parts (e. g. hand washing), foods (e. g., uncooked meat, vegetables, fruits, etc.), cooking utensils, cooking surfaces (e. g., counter tops, sinks, etc.). It has been found that such methods are important in attempts to remove pathogenic microorganisms from human skin as well as other surfaces.

The types of microorganisms which can be found on mammalian skin include viruses, bacteria, and fungi. In general, virologists agree that rhinoviruses, influenza viruses, and adenoviruses are most likely the most relevant viruses which cause respiratory diseases. It is believed that rhinoviruses, in particular, are responsible for acting as the primary cause for the common cold- Rhinoviruses are members of the picornavirus family. As such they are referred to as "naked viruses" since they lack an outer envelope. Such picornaviruses are known to be difficult to inactivate by commonly used means like quaternary ammonium compounds.

Rhinovirus infections are spread from person to person by means of virus-contaminated respiratory secretions. Evidence suggests that the primary mode of transmission is via direct contact, as opposed to inhalation of airborne viral particles. It has been demonstrated that ill persons have a propensity to contaminate their hands and environmental objects. Rhinovirus has been recovered from 40 to 90% of hands of persons experiencing colds and from 6 to 15% of diverse objects. Rhinovirus exhibits good survival on many environmental surfaces for hours after contamination, and infection is readily transmitted by finger-to-finger contact and by finger to contaminated environmental surface if the newly contaminated finger is then used to rub an eye or touch the nasal mucosa.

Since a substantial proportion of rhinovirus colds are transmitted by direct contact from virus contaminated hands or objects, it is possible to lower the risk of acquiring infection by inactivating virus on hands or surfaces. A common household phenol/alcohol disinfectant has been shown to be effecting in disinfecting contaminated environmental surfaces but lacks residual virucidal effects. Hand washing is highly effective at disinfecting contaminated fingers but again suffers from a lack of residual activity.

These shortcomings provide strong opportunities for improved virucidal technologies with residual activity against rhinoviruses.

It has been found that iodine is an effective anti-viral agent and provides residual anti-rhinoviral activity on skin. In experimentally induced and natural cold transmission studies, subjects who used iodine products had significantly fewer colds than placebo users. This indicates that iodine is effective for prolonged periods at blocking the transmission of rhinoviral infections. Thus, the development of hand products, lotions, or washes (without the associated color or odor negatives of iodine) that deliver both immediate and residual anti-viral activity would be effective in reducing the incidents of colds. Likewise, a topical product which exhibits anti-viral activity would be effective in preventing and/or treating virus induced diseases caused by other viruses like adenoviruses, rotaviruses, herpes viruses, respiratory syncytial viruses, coronaviruses, parainfluenza viruses, enteroviruses, influenza viruses, etc..

Examples of such compositions can be found in WO 01/28338, US4,975,217 and WO 01/28556. These compositions typically provide excellent anti-microbial activity when used. However, such topical products typically suffer from other drawbacks in that they are drying to the hands of the typical consumer, they leave a sticky, tacky or palpable residue on the hands or they need rinsing with water in order to remove any residue, and typically do not cover the consumer's hands well enough to provide adequate cleansing without using excessive quantities of the composition. Therefore, there remains a need for providing devices comprising anti-microbial compositions that over-come these drawbacks, and in addition provide portability, economy of use and excellent coverage in use.

### SUMMARY OF THE INVENTION

The present invention provides a device for delivering an anti-microbial composition to the hands of a user, device comprising
i) a liquid anti-microbial composition comprising at least one pyroglutamic acid derivative and from 10% to less than 40% volatile solvent;
ii) a reservoir containing said liquid anti-microbial composition; and
iii) means, in fluid communication with the reservoir, for dispensing said anti-microbial composition as a foam.

The invention is also directed towards providing kits and methods of use. The device of the present invention provides an anti-microbial foam to a user that does not require to be rinsed off by water, and therefore can be used anywhere. The foam further provides an economy of use, enabling a small amount of anti-miorobial composition to be used in providing the foam whose volume is substantially greater than the volume of liquid used to form it, and thereby providing better coverage, and hence a greater degree of sanltization. Furthermore, the use of a foam enabies less residue to remain on the sanitized surface, such as a user's hands.

The present invention further provides methods of disinfecting the hands of a user comprising the steps of:
i- dispensing a liquid anti-microbial composition onto one or both hands of the user from a device according to any one of the preceding claims;
ii- rubbing the user's hands together so as to disperse the foam substantially across the surface of the hands;
iii-continued rubbing of the user's hands for a period of time sufficient for the foam to dissipate such that substantially no liquid residue is left remaining on the hands of said user.

The present invention further provides a kit for disinfecting the hands of a user comprising:
i. a packaged anti-microbial composition comprising at least one pyroglutamic acid derivative;
ii. a reservoir containing said liquid anti-microbial composition; and
iii. means, in fluid communication with the reservoir, for dispensing said anti-microbial composition as a foam.

### DETAILED DESCRIPTION OF THE INVENTION

The present invention is directed towards providing a device for delivering an anti-microdial composition to the hands of a user and uses thereof, the device comprising:
i. a liquid anti-microbial composition comprising at least one pyroglutamic acid derivative and from 10% to less than 40% volatile solvent;
ii. a reservoir containing said liquid anti-microbial composition; and
iii. means, in fluid communication with the reservoir, for dispensing said anti-microbial composition as a foam.

in a further embodiment of the present invention a kit is provided comprising:
i. a packaged anti-microbial composition comprising at least one pyroglutamic acid derivative;
ii. a reservoir suitable containing said anti-microbial composition when in solution; and
iii. means, in fluid communication with the reservoir, for dispensing said anti-microbiel composition as a foam.

As used herein, "anti-microbial" includes anti-viral, anti-microbial, anti-fungel activities, both immediate and residual.

As used herein, "residual anti-viral efficacy" means leaving a residue or imparting a condition on a keratinous tissue (e.g., skin) or other surfaces that remains effective and provides significantanti-viral (specifically against rhinovirus) activity for some time after application. Preferably, the compositions described herein exhibit residual anti-viral efficacy such that a log 1.0 reduction, preferably a log 1.5 reduction, and more preferably a log 2.0 reduction in pathogenic viruses such as rhinovirus is maintained for about 0.5 hours, more preferably for about 1 hour, and most preferably for about 3 hours. The methodology utilized to determine the residual anti-viral efficacy is discussed below in the "Analytical Methods" section.

As used herein, "residual antibacterial efficacy" means leaving a residue or imparting a condition on a keratinous tissue (e. g., skin) or other surfaces that remains effective and provides significant antibacterial (specifically against transient gram positive and negative organisms). Preferably, the compositions described herein exhibit residual anti-bacterial efficacy such that a log 1.0 reduction, preferably a log 1.5 reduction, and more preferably log 2.0 reduction in bacteria such as E. coli is maintained for about 0.5 hours, more preferably for about 1hour, and most preferably for about 3 hours.

As used herein, "safe and affective amount" means an amount of a compound, component, or composition (as applicable) sufficient to signilicantly induce a positive effect (e. g., improvement in dry skin appearance, skin desqus-mation, etc.) but low enough to avoid serious side effects (e. g., undue toxicity or allergic reaction, i. e., to provide a reasonable benefit to risk ratio, within the scope of sound medical judgment.

All percentages and ratios used herein, unless otherwise indicated, are calculated on a weight basis. All percentages are calculated based upon the total composition unless otherwise indicated.

All molar weights are weight average molecular weights and are given in units of grams per mole.

All ingredient levais are in reference to the active level of that ingredient, and are exclusive of solvents, byproducts, or other impurities that may be present in commercially available sources, unless otherwise indicated.

All measurements made are at ambient room temperature, which is approximately 25°C, unless otherwise indicated.

### Anti-Microbial Composition

The device according to the first embodiment of the present invention comprises an anti-microbial composition. When used in the device of the present invention, the anti-microbial composition is preferably a liquid composition. When used in the kit form, the anti-microbial composition may be packaged in any sultable form, such as a liquid, or as a solid that is subsequently dissolved in a solvent such as water before use in the device. Preferably, the anti-microbial composition in both embodiments is a liquid.

The anti-microbial composition of the present invention comprises a safe and effective amount of pyroglutamic acid derivative. As used herein, pyroglutamic acid derivative collectively refers to its stereoisomers and tautomers. Pyroglutamic acid, which is also referred to as pyrrolidone carboxylic acid has two stereoisomers (D and L) and each are preferred for use herein. The D stereoisomer ot pyroglutamic acid is also known by the following names: D-Proline, 5oxo- (+)-2-Pyrrolidone-5-carboxylic acid, (+)-Pyroglutamic acid, (R)-2-Pyrrolidone-5-carboxylic acid, 5- Oxo-D-proline, D-2-Pyrrolidone-5-carboxylic acid, D-Pyroglutamic acid, D-Pyrrolidinonecarboxylic acid, and D-Pyrrolidonecarboxylic acid.

The L stereolsomer of pyroglutamic acid is also known by the following names: L-Proline, 5-oxo (-)-2-Pyrrolidone-5-carboxylic acid, (-)-Pyroglutamic acid, (5S)-2-Oxopyrrolidine-5-carboxylic acid, (S)-(-)-2-Pyrrolidone-5-carboxylic acid, (S)-2-Pyrrolidone-5-carboxylic acid, (S)-5-Oxo-2-pyrrolidinecarboxylic acid, (S)-Pyroglutamic acid, 2-L-Pyrrolidone-5-carboxylic acid, 2-Pyrrolidinone-5-carboxylic acid, 5-Carboxy-2-pyrrolidinone, 5-Oxo-L-proline, 5-Oxoproline, 5-Pyrre-lidinone-2-carboxylic acid. Glutimic acid, Glutiminic acid, L-2-Pyrrolidone-5-carboxylic acid, L-5-Carboxy-2-pyrrolidinone, L-5-Oxo-2- pyrrolidinscarboxylic acid, L-5-Oxoproline, L-Glutamic acid, gamma-lactem, L-Glutimic acid, L-Glutiminic acid, L-Pyroglutamic acid, L-Pyrrolidinonecarboxylic acid, L-Pyrrolidonecarboxylic acid, Oxoproline, PCA, Pidolic acid. Pyroglutamic acid, Pyrrolidinonecarboxylic acid, Pyrrolidone-5-carboxylic acid, and Pyrrolidonecarboxylic acid.

The DL form of pyroglutamic acid (a mixture of the D and L stereoisomers) is known by the following names: DL-Proline, 5-oxo-(+)-2-Pyrrolidone-5-carboxylic acid, ( +)-Pyroglutamic acid, 5-Oxo-DL-Proline, DL-2-Pyrrolidinone-5-carboxylic acid, DL-2-Pyrrolidone-5-carboxylic acid, DLPyroglutamate, DL-Pyroglutamic acid, DL-Pyrrolidonecarboxylic acid, and Oxoproline. The DL form is also commercially available under the tradenama Ajidew A 100.

Some of the above-listed stereolsomers is commercially available form UCIB, France via Bernet Products Corp., New Jersey. Such compounds are sold under trade names like Culvridone (Cu-PCA) and L-FER Pidoiate (Fe-PCA), and Pidolidone.

The compositions of the present invention preferably comprise from 0.01 % to 10%, by weight of the composition, of pyroglutamic acid, more preferably, from 0-1% to 10%, even more preferably from 0.25% to 8%, and most preferably from 1% to 5%.

The anti-microbial composition of the present invention may further comprise ancillary acid materials. These materials contribute to stabilizing the tow pH of the composition, and thereby increase its anti-microbial efficacy. Preferably the compositions herein further comprise an organic acid. Suitable organic acids for use herein include succinic acid, citric acid, malic acid, tartaric acid and mixtures thereof. Preferably, where organic acids are used as ancillary acid materials, the organic acid comprises succinic acid. The compositions herein preferably comprise from 0.1% to 10% ancillary acid, more preferably from 0.1% to 5% anciliary acid by weight of the composition.

The anti-microbial composition of the present invention preferably comprises a liquid carrier material. The preferred liquid carrier materials are aqueous solvents, volatile solvents, or mixtures thereof. Preferred aqueous solvents include water. Where present, water is present in the anti-microbial compositions of the present invention at levels of from 10% to 90%, preferably from 30% to 80% by weight of the composition.

The anti-microbial compositions of the present invention comprise a volatile solvent. Preferred volatile solvents comprise monohydric linear and branched C₁ to C₆ alcchols or mixtures thereof, more preferably ethanol- Preferably the anti-microbial compositions of the present invention comprise a volatile solvent. This is advantageous to enable the composition to have substantial anti-microbial efficacy, and also to enable it to evaporate quickly from the surface to which it is applied leaving little or substantially no residue. It is has been found that such compositions are useful for cleansing the hands and other surfaces without the requirement for rinsing with water, and can therefore be used anywhere. The volatile solvents are present at levels of from 10% to lass than 40%, more preferably from 10% to 25%. The compositions herein require volatile solvents such as ethanol to present at the above levels in order to provide improved immediate anti-microbial activity. However, at levels of 40% and above, the volatile solvents tand to reduce the structure of the foam produced by the device of the present invention, and thereby reduce the area able to be covered by the product. Without wishing to be bound by theory it is beileved that levels of volatile solvent above those claimed herein reduce the structure of the foam composition by lowering the surface tension of the liquid composition.

In order for the anti-microbial composition to provide a substantive foam, and also to provide effective cleansing of the user's hands or other surfaces, it is preferable for the anti-microbial composition to comprise a surfactant. Suitable surfactants include anionic, cationic, non-ionic, zwitterionic or mixtures thereof, preferably anionic, nonionic or mixtures thereof, more preferably anionic. Preferably the anti-microbial composition herein comprises from 0.1% to 25% surfactant.

Non-limiting examples of anionic lathering surfactants include those selected from the group consisting of alkyl and alkyl ether sultates, sulfated monoglycerides, sulfonated olefins, alkyl aryl sulfonates, primary or secondary alkane sulfonates, alkyl sulfosuccinates, acyl taurates, acyl isothlonatas, alkyl glycarylather sulfonate, sulfonated methyl esters, sulfonated fatly acids, alkyl phosphates, acyl glutamsies, alkyl sulfoacetates, acylated peptides, alkyl ether carboxylates, acyl lactylates, anionic fluorosurfactants, or mixtures thereof. Mixtures of anionic surfactants can be used effectively in the present invention. Preferred anionic surfactants for use in the antimicrobial compositions include alkyl and alkyl ether sulfates. These materials have the respective formulae RIO-SOSM and R1 (CH2H40) x-O-SO3M, wherein R1 is a saturated of unsaturated, branched or unbranched alkyl group from about 8 to about 24 carbon atoms, x is 1 to 10, and M is a water-soluble cation such as ammonium, sodium, potassium, magnesium, triethanolamine, diethanolamine and monoethanolamine. The alkyl sulfates are typically made by the sulfation of monohydric alcohols (having from about 8 to about 24 carbon atoms) using sulfur trioxide or other known sulfation technique. The alkyl ether sulfates are typically made as condensation products of ethylene oxide and monohydric alcohols (having from about 8 to about 24 carbon atoms) and then sulfated. Non-limiting examples include ammonium lauryl sulfate, sodium lauryl sulfate, preferably ammonium lauryl sulfate. Preferably, the compositions of the present invention comprise from 0.1% to 10% anionic surfactant, more preferably from 0.1 % to 5% by weight of the composition.

Preferably, the anti-microbial compositions herein further comprise a nonionic surfactant Non-ionic surfactants are useful herein in providing good foaming characteristics, and to enable the solubilisation of the saticylic acid derivative. Non-limiting examples of suitable nonionic surfactants for use herein include the ethoxylated alcohols such as polyox-yethoxylated isohexadecyl ether available under the tradename. Arlasolve 200 from Uniqema (ICI Surfactants).

Preferably, the anti-microblal compositions of the present invention are buffered to a pH of from 2 to 5. Without wishing to be bound by theory it is believed that the virucidavvirustatic properties of the compositions herein are enhanced when the composition is maintained at a low pH. The low pH is believed to protonate the viral capsule and therefore inactivate the virus and prevent infection.

The anti-microbial compositions of the present invention preferably comprise, in total, 30% or less non-voistile ingredients. Such ingredients include those ingredients referred to above such as the salicylic acid derivatives, PCA-derivatives, surfactants and the like, and further include optional, non-volatile ingredients such as molsturizers, preservatives, emoilients, colours, fragrances, sunscreens and the like known to those skilied in the art. More preferably the levels of such non-volatile are 10% or less by weight of the composition. Without wishing to be bound by theory, it is believed that by minimizing the levels of non-volatile components in the anti-microbial compositions the residue left on the hands of a user is minimized so that is practically imperceptible, and therefore does not require rinsing of the hands. The compositions of the present invention are preferably leave-on products. As used herein, "leave-on" means products that can be applied to the skin of a consumer, and left in contact with the skin following application. The leave-on products herein do not need rinsing off with water, or removal via a tissue or other implement.

The present invention is further directed to an anti-microbial composition in the form of a foam suitable for dispensing from the device according to the present invention having a density of less than 0.4 g/cm³, preferably less than 0.3 g/cm³, more preferably less than 0-2 g/cm³.

### Foaming Device

According to the preferred embodiments of the present invention, the device for delivering an anti-microbial composition comprises a reservoir and means for dispensing the anti-microbial composition as a foam or foaming composition. As used herein, "loam" and "foaming composition" mean compositions in which a liquid or gel is mixed with a gas such as an aerosol propellant or ambient air to form a semi-structured composition or mass comprising bubbles of gas trapped within a liquid or gel matrix.

The device herein comprises a reservoir suitable for holding and storing the anti-microbial composition during transport and use. Such reservoir may be releasably attached to the dispensing apparatus by means such as screw threads, latches, hinges, clips or other such suliable means, or integrally molded thereto. The reservoir may comprise a rigid body, or alternatively may be comprised of a non-rigid body such that the reservoir collapses in on itself during use. Any suitable material may be used to form the reservoir. Suitable non-limiting examples include Polyethylene Tetrephthalete (PET), high density polyethylene (HDPE), polypropylene (PP) or mixtures thereof.

The apparatus for dispensing the anti-microbial composition comprises an outlet in fluid communication with a reservoir where the anti-microbial composition is stored, through which the anti-microbial composition is ejected for us, and may comprise a pump, a squeeze bottle, an aerosol, or other such similar apparatus known to the person skilled in the art. The apparatus for dispensing is such that the user can dispense the anti-microbial composition by operating the dispensing apparatus. For example, a non-aerosol pump can be operated by depressing the top of the pump so as to force the anti-microbial composition out, or alternatively using an aerosol, depressing a valve so as to allow the propeilant gases in the pressurized container to eject the anti-microbial composition Preferably, the apparatus for dispensing comprises a pump, more preferably a non-aerosol pump spray

The apparatus according to the present invention further comprises means for mixing said anti-microbial composition with a gas either before of during dispensing so as to form a foam. Preferably the means for mixing allow the anti-microbial composition and gas to be mixed at a volume ratio of at least 1:1 (anti-microbial composition:gas), preferably from 1:1 to 1:30, more preferably at a volume ratio of from 1:5 to 1:15. The gas preferably is comprised of ambient air, propellant gas, or mixtures thereof.

Non-limiting examples of suitable means for dispensing the anti-microblai formulation as a foam are described in US6053364, EP835500A1, EP613728A1 and WO 04/078359A1. Briefly, an apparatus for dispensing an air-liquid mixture, in particular a foam comprising an operating unit, which unit comprises at least an air pump and a liquid pump, which are essentially concentric, and each comprise a piston chamber with a piston which is displaceable therein, while each pump comprises an inlet and a discharge, and an operating componant is present for operating the two pumps. which operating component is integral with the piston of the liquid pump, and therein comprises an outflow channel connected to the outlet, which channel can be connected to the discharge of the pumps, while shut-off means, which make it possible to suck up air or liquid, respectively, into the appropriate piston chambers and to dispense these fluids to the outflow channel, are present in the inlet and discharge of the air and liquid pump, the connection between the outflow channel and the relevent pumps being interrupted while air or liquid is being sucked up, and the air pump comprises a double-acting shut-off device, which shuts off both the inlet of air to the air pump and shuts off the discharge of air therafrom.

The device of the present invention may be supplied as a kit comprising:
i. a packaged anti-microbial composition comprising at least one pyroglutamic acid derivative;
ii. a reservoir containing said liquid anti-microbial composition: and
iii. means, in fluid communication with the reservoir, for dispensing said anti-microbial composition as a foam.

The device and kit of the present invention may be used in a method of cleansing a user's hands comprising the steps of:
i. dispansing a liquid anti-microbial composition onto one or both hands of the user from a device as describedherein;
ii. rubbing the user's hands together so as to disperse the foam substantially across the surface of the hands; and
iii. continue rubbing of the user's hands for a period of time sufficient for the foam to dissipate such that substantially no liquid residue is left remaining on the hands of said user.

### Examples

The followinig are non-limiting examples of compositions that may be packaged into packages that for the devices according to the present invention. The following anti-microbial compositions are all packaged in an F2-Finger Pump Foamer (code F2-L11) available from Airspray. When actuated, this package dispenses approximately 0.8ml of composition at a 1:10 ratio with air to provide a foam having a density of about 0.1 g/cm³. The compositions are effective hand sanitizers, having both immediate and residual anti-bacterial and virustatic activity. Substantially little or no residue was left upon the hands following continued rubbing of the hands and evaporation of the volatile solvents.

| **Ingredient** | **Ex.1** | **Ex.2** | **Ex. 3** |
|---|---|---|---|
| Ethanol | 20% | 25 | 15% |
| Succinic Acid | 1% | 0.5% | 2% |
| PCA | 3.5% | 3% | 4% |
| Ammonium lauryl sulfate | 1% | 2% | 4% |
| Arlascive 200¹ | 1% | - | 0.5% |
| Tesobetaine | - | 1 % | 0-5% |
| Proylene Glycol | - | 5% | - |
| Water | Q.S. to 100% | Q.S. to 100% | Q.S. to 100% |
| ¹POE (20) Isohexadecyl ether, available from Uniqama. | | | |

## Claims

1. A device for delivering an anti-microbial composition to the hands of a user, the device comprising
i. a liquid anti-microbial composition comprising at least one pyroglutamic add derivative and from 10% w/w to less than 40% w/w volatile solvent;
ii. a reservoir containing said liquid anti-microbial composition; and
iii. means, in fluid communication with the reservoir, for dispensing said anti-microbial composition as a foam.

2. A device according to claim 1 wherein the anti-microbial composition is anti-bacterial, anti-viral or mixtures thereof.

3. A device according to claim 1 or claim 2 wherein said volatile solvent comprises a C₁ to C₆ alcohol, preferably ethanol.

4. A device according to claim 3 wherein said liquid anti-microbial composition comprises from 10% w/w to 25% w/w volatile solvent.

5. A device according to any one of the precading claims wherein said anti-microbial composition comprises an ancillary acid, preferably an organic acid, more preferably succinic acid.

6. A device according to any one of the preceding claims wherein said liquid anti-microbial composition comprises a surfactant, preferably an anionic surfactant.

7. A device according to claim 7 wherein said surfactant is present at levels of from 0.1% w/w to 25% w/w

8. A device according to any one of the preceding claims wherein the liquid anti-microbial composition has a pH of from 2 to 5.

9. A device according to any one of the preceding claims wherein said liquid anti-microbial composition comprises 30% w/w or less non-volatile ingredients, preferably 10% w/w or less.

10. A device according to any one of the preceding claims wherein said liquid anti-microbial composition comprises from 0.1% w/w to 10% w/w pyroglutamic acid derivative.

11. A device according to any one of the preceding claims wherein said means for dispensing the anti-microbial composition mixes the liquid anti-microbial composition and the gas at a volume ratio of at least 1:1, preferably at a volume ratio of from 1:1 to 1:30, more preferably at a volume ratio of from 1:5 to 1:15.

12. A method of disinfecting the hands of a user comprising the steps of:
i. dispensing a liquid anti-microbial composition onto one or both hands of the user from a device according to any one of the preceding claims;
ii. rubbing the user's hands together so as to disperse the foam substantially across the surface of the hands;
iii. continued robbing of the user's hands for a period of time sufficient for the foam to dissipate such that substantially no liquid residue is left remaining on the hands of said user.

13. The device according to claim 1 in the form of a kit.

## Patentansprüche

1. Vorrichtung zum Abgeben einer antimikrobiellen Zusammensetzung an die Hände eines Benutzers, wobei die Vorrichtung Folgendes umfasst
i. eine flüssige antimikrobielle Zusammensetzung, umfassend mindestens ein Pyroglutaminsäurederivat und von 10 Gew.-% bis weniger als 40 Gew.-% flüchtiges Lösungsmittel;
ii. einen Vorratsbehälter, der die flüssige antimikrobielle Zusammensetzung enthält; und
iii. Mittel, die in Fuidverbindung mit dem Vorratsbehälter sind, für die Abgabe der antimikrobiellen Zusammensetzung als Schaum.

2. Vorrichtung nach Anspruch 1, wobei die antimikrobielle Zusammensetzung antibakteriell, antiviral oder Mischungen davon ist.

3. Vorrichtung nach Anspruch 1 oder Anspruch 2, wobei das flüchtige Lösungsmittel einen C₁- bis C₆-Alkohol, vorzugsweise Ethanol, umfasst.

4. Vorrichtung nach Anspruch 3, wobei die flüssige antimikrobielle Zusammensetzung von ungefähr 10 Gew.-% bis ungefähr 25 Gew.-% flüchtiges Lösungsmittel umfasst.

5. Vorrichtung nach einem der vorstehenden Ansprüche, wobei die antimikrobielle Zusammensetzung eine zusätzliche Säure, vorzugsweise eine organische Säure, mehr bevorzugt Bernsteinsäure, umfasst.

6. Vorrichtung nach einem der vorstehenden Ansprüche, wobei die flüssige antimikrobielle Zusammensetzung ein Tensid, vorzugsweise ein anionisches Tensid, umfasst.

7. Vorrichtung nach Anspruch 7, wobei das Tensid in Konzentrationen von 0,1 Gew.-% bis 25 Gew.-% vorhanden ist.

8. Vorrichtung nach einem der vorstehenden Ansprüche, wobei die flüssige antimikrobielle Zusammensetzung einen pH von 2 bis 5 hat.

9. Vorrichtung nach einem der vorstehenden Ansprüche, wobei die flüssige antimikrobielle Zusammensetzung zu 30 Gew.-% oder weniger nichtflüchtige Bestandteile umfasst, vorzugsweise zu 10 Gew.-% oder weniger.

10. Vorrichtung nach einem der vorstehenden Ansprüche, wobei die flüssige antimikrobielle Zusammensetzung von 0,1 Gew.-% bis 10 Gew.-% Pyroglutaminsäurederivat umfasst.

11. Vorrichtung nach einem der vorstehenden Ansprüche, wobei das Mittel zur Abgabe der antimikrobiellen Zusammensetzung die flüssige antimikrobielle Zusammensetzung und das Gas in einem Volumenverhältnis von mindestens 1:1, vorzugsweise in einem Volumenverhältnis von 1:1 bis 1:30, mehr bevorzugt in einem Volumenverhältnis von 1:5 bis 1:15 mischt.

12. Verfahren zum Desinfizieren der Hände eines Benutzers, das die folgenden Schritte umfasst:
i. Abgabe einer flüssigen antimikrobiellen Zusammensetzung aus einer Vorrichtung nach einem der vorstehenden Ansprüche auf eine oder beide Hände des Benutzers;
ii. Reiben der Hände des Benutzers aneinander, um den Schaum im Wesentlichen über die Oberfläche der Hände zu verteilen;
iii. weiteres Reiben der Hände des Benutzers für einen ausreichenden Zeitraum, damit sich der Schaum dissipiert, so dass im Wesentlichen kein flüssiger Rückstand auf den Händen des Benutzers zurückgelassen wird.

13. Vorrichtung nach Anspruch 1 in der Form eines Sets.

## Revendications

1. Dispositif pour distribuer une composition antimicrobienne sur les mains d'un utilisateur, le dispositif comprenant
i. une composition antimicrobienne liquide comprenant au moins un dérivé d'acide pyroglutamique et de 10 % m/m à moins de 40 % m/m de solvant volatil ;
ii. un réservoir contenant ladite composition antimicrobienne liquide ; et
iii. un moyen, en communication du point de vue des fluides avec le réservoir, pour distribuer ladite composition antimicrobienne en tant que mousse.

2. Dispositif selon la revendication 1, dans lequel la composition antimicrobienne est antibactérienne, antivirale ou leurs mélanges.

3. Dispositif selon la revendication 1 ou la revendication 2, dans lequel ledit solvant volatil comprend un alcool en C₁ à C₆, de préférence de l'éthanol.

4. Dispositif selon la revendication 3, dans lequel ladite composition antimicrobienne liquide comprend de 10 % m/m à 25 % m/m de solvant volatil.

5. Dispositif selon l'une quelconque des revendications précédentes, dans lequel ladite composition antimicrobienne comprend un acide secondaire, de préférence un acide organique, plus préférablement de l'acide succinique.

6. Dispositif selon l'une quelconque des revendications précédentes, dans lequel ladite composition antimicrobienne liquide comprend un agent tensioactif, de préférence un agent tensioactif anionique.

7. Dispositif selon la revendication 7, dans lequel ledit agent tensioactif est présent à des taux allant de 0,1 % m/m à 25 % m/m.

8. Dispositif selon l'une quelconque des revendications précédentes dans lequel la composition antimicrobienne liquide a un pH allant de 2 à 5.

9. Dispositif selon l'une quelconque des revendications précédentes, dans lequel ladite composition antimicrobienne liquide comprend 30 % m/m ou moins d'ingrédients non volatils, de préférence 10 % m/m ou moins.

10. Dispositif selon l'une quelconque des revendications précédentes, dans lequel ladite composition antimicrobienne liquide comprend de 0,1 % m/m à 10 % m/m de dérivé d'acide pyroglutamique.

11. Dispositif selon l'une quelconque des revendications précédentes, dans lequel ledit moyen pour distribuer la composition antimicrobienne mélange la composition antimicrobienne liquide et le gaz à un rapport en volume d'au moins 1:1, de préférence à un rapport en volume allant de 1:1 à 1:30, plus préférablement à un rapport en volume allant de 1:5 à 1:15.

12. Procédé de désinfection des mains d'un utilisateur comprenant les étapes consistant à :
i. distribuer une composition antimicrobienne liquide sur l'une ou sur les deux mains de l'utilisateur à partir d'un dispositif selon l'une quelconque des revendications précédentes ;
ii. frotter les mains de l'utilisateur conjointement de façon à disperser la mousse essentiellement à travers la surface des mains ;
iii. continuer de frotter les mains de l'utilisateur pendant une période de temps suffisante pour que la mousse se dissipe de telle sorte qu'il ne reste essentiellement plus de résidu liquide sur les mains dudit utilisateur.

13. Dispositif selon la revendication 1, sous la forme d'une trousse.
